# FASCICULE DE BREVET EUROPEEN

(11) **EP 1 923 012 B1**
(45) Date de publication et mention de la délivrance du brevet: **16.06.2010**
(21) Numéro de dépôt: 07356161.5
(22) Date de dépôt: 15.11.2007
(51) Int. Cl.: A61B 17/72, A61B 17/16

(54) **Implant d'arthrodèse inter-phalangienne, kit chirurgical et procédé de fabrication correspondants**
Implantat für die interphalangäre Arthrodese, entsprechender chirurgischer Teilesatz und Herstellungsverfahren
Interphalangeal arthrodesis implant, corresponding surgical kit and manufacturing method

(30) Priorité: 16.11.2006 FR 0610043
(43) Date de publication de la demande: 21.05.2008
(73) Titulaire: Newdeal, 69006 Lyon (FR)
(72) Inventeur: Coilard-Lavirotte, Jean-Yves, Paul, Albert, 69450 Saint Cyr au Mont d'Or (FR); Laffenetre, Olivier, 33000 Bordeaux (FR)
(74) Mandataire: Martin, Didier Roland Valéry

(56) Documents cités:
- EP-A- 1 582 162
- WO-A-03/007830
- WO-A-2005/039425
- WO-A-2005/096975
- FR-A1- 2 778 082
- FR-A1- 2 787 313
- US-A- 4 522 200
- US-A- 5 713 904
- US-A1- 2005 283 159

## Description

La présente invention se rapporte aux implants médicaux destinés à être fixés dans les os lors d'interventions relevant de la chirurgie orthopédique, et notamment aux implants utilisés pour assembler des os ou des fragments d'os en vue d'obtenir leur jonction par fusion osseuse.

La présente invention concerne plus particulièrement un implant médical destiné à être interposé entre un premier os et un second os afin de maintenir lesdits premier et second os sensiblement accolés l'un à l'autre en vue d'obtenir la fusion osseuse de ces derniers, ledit implant présentant au moins un premier tronçon conçu pour être introduit dans le premier os et pourvu d'un premier moyen de fixation agencé pour retenir ledit implant au sein du premier os, ainsi qu'un second tronçon conçu pour être introduit dans le second os et pourvu d'un second moyen de fixation agencé pour retenir ledit implant au sein du second os.

La présente invention concerne également un kit chirurgical.

La présente invention concerne enfin un procédé de fabrication d'un implant médical pourvu d'un moyen de fixation agencé pour retenir ledit implant au sein d'un os.

Dans le domaine de la chirurgie orthopédique, de nombreuses interventions ont pour objet la fixation d'un tissu à un autre en vue par exemple de faciliter la prise d'un greffon, de restaurer un lien naturel entre tissus après que ledit lien a été endommagé ou détruit par une maladie ou un traumatisme, de consolider un os ou encore de renforcer une articulation fragilisée.

En particulier, il est connu de pratiquer chez des patients souffrant d'importantes lésions articulaires, notamment d'arthrose sévère ayant conduit à une dégradation des surfaces articulaires cartilagineuses et/ou osseuses, une arthrodèse, c'est-à-dire de procéder à l'immobilisation forcée de l'articulation concernée en provoquant une fusion osseuse entre les os qui la constituent.

L'arthrodèse peut par exemple être pratiquée au niveau des articulations inter-phalangiennes, notamment du pied, et faire appel à différentes techniques chirurgicales.

L'une de ces techniques chirurgicales consiste à réséquer les extrémités des deux phalanges consécutives que l'on souhaite faire fusionner, de manière à créer des surfaces dites «*saignantes*», à placer les surfaces saignantes obtenues au contact l'une de l'autre, puis à mettre en place un implant de fixation destiné à faciliter régénération des tissus osseux à l'interface de contact.

A cet effet, il est connu d'introduire une broche, c'est-à-dire une fine tige métallique cylindrique, selon l'axe médullaire desdites phalanges de telle sorte que ladite broche traverse l'articulation de part en part et limite le débattement radial d'une phalange par rapport à l'autre.

Une variante de cette technique consiste à utiliser une vis plutôt qu'une broche, ladite vis étant insérée depuis l'extrémité du doigt selon l'axe médullaire de la phalange distale de manière à traverser l'articulation et à maintenir les deux phalanges comprimées l'une contre l'autre.

Bien qu'ils donnent généralement satisfaction sur le plan thérapeutique, de tels implants d'arthrodèse souffrent cependant d'inconvénients non négligeables liés notamment à la qualité de la liaison mécanique qu'ils réalisent entre les tissus, et plus particulièrement entre les os, qu'ils ont pour fonction de maintenir.

En effet, les implants sus-mentionnés n'assurent qu'un maintien mécanique partiel du premier os contre le deuxième, plus particulièrement de la première phalange par rapport à la seconde, ce qui tend à ralentir et à compliquer la reconstitution d'un tissu osseux à l'interface de contact desdits os.

Plus précisément, lorsque le patient déplace le membre concerné, par exemple lors de la marche s'il s'agit d'une arthrodèse réalisée au niveau du pied, les contraintes mécaniques exercées sur l'articulation peuvent être à l'origine de mouvements relatifs parasites desdits os, lesquels sont susceptibles d'entraîner l'arrachement et l'abrasion du tissu en construction.

A titre d'exemple, les os sont susceptibles de coulisser le long de la broche, donc de se séparer axialement l'un de l'autre, ou encore de pivoter autour de l'axe formé par ladite broche. De même, une vis médullaire peut se desserrer sous l'effet d'une rotation relative du premier os par rapport au second en autorisant un débattement relatif desdits os.

En outre, les implants d'arthrodèse de l'art antérieur exposent généralement le patient à un traumatisme des tissus de l'articulation situés au voisinage de la zone traitée. Ainsi, une broche médullaire est susceptible de se déplacer longitudinalement jusqu'à venir s'appuyer contre les tissus corticaux ou les tissus mous situés dans le prolongement de son axe d'extension, voire de perforer lesdits tissus. La saillie formée par la tête de vis est quant à elle de nature à venir au contact des tissus articulaires environnants et ainsi former un point de compression douloureux et/ou provoquer une usure prématurée desdits tissus.

Ensuite, la position forcée dans laquelle les implants d'arthrodèse de l'art antérieur bloquent l'articulation diffère fréquemment de l'anatomie naturelle du patient, ce qui génère une sensation d'inconfort, voire des douleurs, chez ce dernier.

Enfin, les implants d'arthrodèse de l'art antérieur nécessitent généralement une préparation chirurgicale importante de l'articulation et présentent de surcroît un encombrement significatif, ce qui tend à rendre l'intervention relativement longue, complexe et traumatisante pour le patient.

On connaît par ailleurs, par les documents FR-2 787 313, WO-2005/039425, US-5,713,904, WO-03/007830 et US-2005/0283159, divers implants destinés à être déployés après qu'ils ont été introduits dans un logement percé dans l'os.

On connaît notamment par le document FR-2 787 313 un implant conforme au préambule de la revendication 1 réalisé dans un matériau à mémoire de forme.

Les objets assignés à la présente invention visent par conséquent à remédier aux inconvénients susmentionnés et à proposer un nouvel implant médical qui puisse se fixer de façon solide et stable dans un os.

Un autre objet de la présente invention vise à proposer un nouvel implant médical destiné à être fixé dans un os qui soit particulièrement facile à mettre en oeuvre.

Un autre objet de l'invention vise à proposer un nouvel implant médical de forme simple et compacte présentant un faible encombrement.

Un autre objet de la présente invention vise à proposer un nouvel implant médical qui soit particulièrement peu onéreux à fabriquer.

Un autre objet de la présente invention vise à proposer un nouvel implant médical qui soit particulièrement atraumatique et qui présente un réel confort d'utilisation pour le patient.

Les objets assignés à l'invention visent également à proposer un nouveau kit chirurgical présentant une grande facilité d'utilisation et permettant une mise en oeuvre rapide, simple et sûre d'un implant conforme à l'invention.

Un autre objet assigné à l'invention vise à proposer un nouveau procédé de fabrication d'un implant médical permettant de doter ledit implant d'un moyen de fixation dans l'os qui soit sûr et efficace.

Enfin, un autre objet de l'invention vise à proposer un nouveau procédé de fabrication d'un implant médical qui soit particulièrement simple et économique à mettre en oeuvre.

Les objets assignés à l'invention sont atteints à l'aide de l'implant médical défini dans la revendication 1 annexée.

Les objets assignés à l'invention sont également atteints à l'aide d'un kit chirurgical caractérisé en ce qu'il comprend :
- d'une part un implant médical conforme à l'invention,
- et d'autre part un instrument chirurgical destiné à la préparation osseuse, comme défini dans la revendication 22..

Les objets assignés à l'invention sont enfin atteints à l'aide du procédé de fabrication d'un implant médical conforme à invention, défini dans la revendication 23 annexée.

D'autres objets, caractéristiques et avantages de l'invention apparaîtront plus en détails à la lecture de la description qui suit, ainsi qu'à l'aide des dessins annexés, fournis à titre illustratif et non limitatif, parmi lesquels :
- La figure 1 illustre, selon une vue en perspective, une variante de réalisation d'un implant conforme à l'invention.
- La figure 2 illustre, selon une vue en perspective, une variante de réalisation d'un implant conforme à l'invention mise en oeuvre dans le cadre d'une arthrodèse inter-phalangienne.
- La figure 3 illustre, selon une vue de côté, l'implant de la figure 1.
- La figure 4 illustre, selon une vue de côté, une autre variante de réalisation d'un implant conforme à l'invention et schématise son introduction dans les os.
- La figure 5A illustre, selon une vue en perspective, une portion d'un instrument chirurgical destiné à un kit chirurgical conforme à l'invention.
- La figure 5B illustre la section transversale d'une empreinte pouvant être marquée dans un os à l'aide de l'instrument illustré sur la figure 5A.
- La figure 6 illustre, selon une vue de dessus, une variante de réalisation d'un implant conforme à l'invention.
- La figure 7 illustre, selon une vue en perspective, l'implant de la figure 6.
- La figure 8 illustre, sur une même vue de face, les sections transversales A-A et B-B de l'implant représenté sur la figure 6, rapportées dans un même plan.

L'implant médical 1 conforme à l'invention, ci-après «*implant*», est destiné à être introduit dans le corps d'un patient humain ou animal afin de réaliser la fixation d'un tissu avec un autre tissu.

Plus précisément, l'implant 1 est destiné à être interposé entre un premier os 2 et un second os 3 afin de maintenir lesdits premier et second os 2, 3 sensiblement accolés l'un à l'autre en vue d'obtenir la fusion osseuse de ces derniers.

Ledit implant 1 constitue donc de préférence un implant d'arthrodèse, bien que l'on puisse envisager son utilisation dans le cadre d'autres interventions, telles que la réduction et la consolidation de fractures.

En outre, l'implant 1 constituera de préférence un implant intra-médullaire, notamment destiné à être engagé dans la diaphyse et/ou l'apophyse du premier os (respectivement du second) selon une direction proche de l'axe médullaire de ce dernier.

Au sens de l'invention, le premier et/ou le second os peuvent indifféremment être naturellement présents dans la zone traitée ou rapportés sous forme de greffons, endogènes ou exogènes.

De façon particulièrement préférentielle, le premier os 2 et le second os 3 sont des phalanges, l'implant 1 constituant alors un implant d'arthrodèse inter-phalangienne. Plus spécifiquement, ledit implant est de préférence conçu pour être implanté dans le pied d'un patient et supporter les contraintes mécaniques liées à la marche.

L'implant 1 conforme à l'invention présente au moins un premier tronçon 4 conçu pour être introduit dans le premier os 2 et pourvu d'un premier moyen de fixation 5 agencé pour retenir ledit implant 1 au sein du premier os 2.

Selon l'invention, l'implant 1 présente également un second tronçon 6 conçu pour être introduit dans le second os 3 et pourvu d'un second moyen de fixation 7 agencé pour retenir ledit implant 1 au sein du second os 3.

Selon une caractéristique importante de l'invention, le premier moyen de fixation 5 comporte une pluralité de branches d'ancrage 10 solidaires du premier tronçon 4 et agencées selon une disposition non coplanaire de sorte à créer une liaison d'encastrement entre ledit implant 1 et ledit premier os 2.

Par «*liaison d'encastrement*», on désigne une liaison cinématique dans laquelle tous les degrés de liberté, c'est-à-dire les trois degrés de liberté en translation et les trois degrés de liberté en rotation, sont supprimés. En d'autres termes, le premier moyen de fixation 5 est conçu pour assujettir l'implant 1 au premier os 2 de sorte que ces derniers ne forment plus qu'un même ensemble sur le plan cinématique.

Par «*disposition non coplanaire*»*,* on indique que les branches d'ancrage 10 conformes à l'invention forment un réseau qui s'étend selon les trois dimensions de l'espace. En effet, si les branches d'ancrage s'étendaient sensiblement selon un même plan de l'espace, c'est-à-dire occupaient une région de l'espace inscrite dans un parallélépipède rectangle dont la longueur et/ou la largeur sont nettement supérieures à son épaisseur, la fixation risquerait de manquer de stabilité, un jeu entre l'implant 1 et l'os 2 étant susceptible d'apparaître et/ou une déformation en flexion du premier moyen de fixation 5 selon un axe contenu dans ledit plan étant susceptible de se produire (ledit premier moyen de fixation 5 tendant à «*plier*» sous la contrainte).

Il est envisageable que la disposition non coplanaire des branches d'ancrage 10 soit obtenue à l'aide de deux branches d'ancrage recourbées de sorte à présenter, à elles deux, au moins trois segments de longueur suffisante et de directions divergentes deux à deux. Toutefois, une telle solution risquerait de compliquer la mise en place de l'implant 1. C'est pourquoi le premier moyen de fixation 5 comporte de préférence au moins trois, et de façon particulièrement préférentielle quatre, branches d'ancrage 10.

Avantageusement, la multiplication des points d'arrimage ainsi créés par les branches d'ancrage permet d'améliorer l'assise de l'implant dans l'os et par conséquent la stabilité de la fixation, chaque branche d'ancrage 10 jouant à l'égard du réseau formé par les autres branches d'ancrage 10 le rôle d'une jambe de force qui contribue à rigidifier ladite fixation.

Par commodité de description, on fera référence dans ce qui suit aux branches d'ancrage 10 dans leur ensemble, bien qu'il soit envisageable que chaque branche d'ancrage 10 fasse l'objet indépendamment de variations de réalisation selon une combinaison quelconque de l'une ou l'autre des caractéristiques détaillées ci-dessous.

Selon l'invention, les branches d'ancrage 10 sont «*solidaires du premier tronçon 4*», c'est-à-dire qu'elles se trouvent fixées en liaison d'encastrement avec ledit premier tronçon 4. Au sens de l'invention, il n'existe donc ni jeu, ni débattement possible, autrement que par une éventuelle déformation intrinsèque des matériaux sous la contrainte, des branches d'ancrage 10 par rapport au premier tronçon 4 auquel elles sont rattachées.

Il est remarquable que cette liaison intime des branches d'ancrage 10 avec le premier tronçon 4 préexiste avant la mise en place de l'implant 1, et plus précisément avant l'introduction du premier tronçon 4 dans le premier os 2. En d'autres termes, lesdites branches d'ancrage 10 sont conçues pour être mises en oeuvre concomitamment à l'introduction du premier tronçon 4 dans le premier os 2, et non rapportées ultérieurement à cette opération d'introduction.

Selon une variante de réalisation préférentielle, les branches d'ancrage 10 sont venues de matière avec le premier tronçon 4.

De préférence, les branches d'ancrage 10 sont réparties en corolle autour du premier tronçon 4. Avantageusement, une telle répartition, de préférence sensiblement homogène, permet d'équilibrer la portée d'appui de l'implant 1 contre l'os 2 et d'améliorer la stabilité de la fixation.

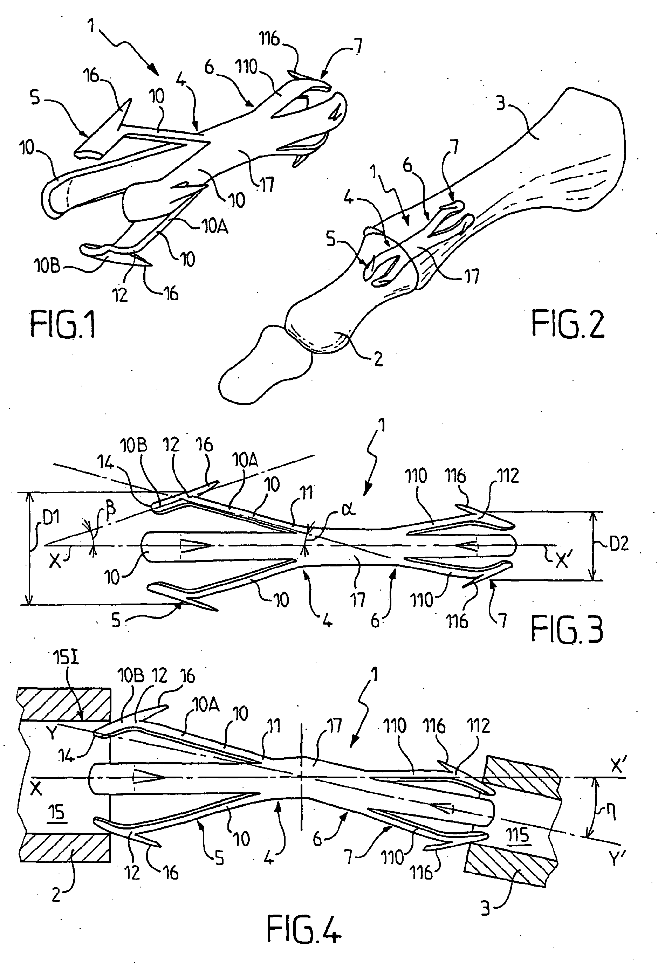

Bien qu'il soit envisageable que le premier moyen de fixation 5 soit positionné en une zone quelconque de l'implant 1, le premier tronçon 4 est de préférence situé à une extrémité de l'implant 1, et les branches d'ancrage 10 s'étendent de préférence à partir dudit premier tronçon 4 et sensiblement au-delà de ladite extrémité, tel que cela est illustré sur les figures 1 à 4.

En d'autres termes, les branches d'ancrage 10 sont de préférence situées en bout de l'implant 1 et sensiblement dirigées vers l'extérieur dudit implant. En particulier, si le premier tronçon 4 forme l'extrémité distale de l'implant 1, les branches d'ancrage pointent de préférence depuis le premier tronçon 4 vers une direction sensiblement opposée à l'extrémité proximale de l'implant.

De préférence, le premier tronçon 4, et plus généralement l'implant 1 dans son ensemble, est sensiblement oblong.

Bien qu'il soit envisageable, selon une variante non conforme à l'invention, que les branches d'ancrage soient contenues dans une enveloppe virtuelle formée par le prolongement du premier tronçon 4 au-delà de l'extrémité à laquelle ledit premier tronçon est situé, les branches d'ancrage 10 s'étendent de préférence vers l'extérieur au-delà de ladite enveloppe afin de maximiser l'étendue de l'ancrage dans l'os, c'est-à-dire la portée du premier moyen de fixation 5.

Plus précisément, tel que cela est illustré sur les figures 1 à 4, le premier tronçon s'étendant de préférence selon un premier axe d'extension (XX'), les branches d'ancrage 10 comportent une portion divergente 10A qui diverge dudit premier axe d'extension (XX') à partir dudit premier tronçon 4.

En d'autres termes, l'agencement spatial de la matière constitutive des branches d'ancrage 10 comprend au moins une composante d'extension radiale centrifuge par rapport au premier axe d'extension (XX'), de telle sorte que les portions divergentes 10A s'ouvrent vers l'extérieur par rapport au premier tronçon 4.

L'angle d'ouverture α de la portion divergente par rapport au premier axe d'extension (XX') est de préférence sensiblement compris entre 5° et 20°.

Bien que les branches d'ancrage 10 puissent être formées par des plaques sécantes sensiblement jointives (réunies par exemple selon un assemblage cruciforme), lesdites branches sont de préférence longilignes et disjointes, une portion de base 11 les rattachant au premier tronçon 4. En outre, bien que lesdites branches d'ancrage 10 puissent être ramifiées, elles sont de préférence sensiblement linéaires. Un tel agencement favorise notamment la pénétration desdites branches dans l'os.

En outre, les branches d'ancrage 10 pourront être étagées le long du premier tronçon 4, par exemple en épi ou en chevrons, ou encore être issues d'une même portion de base 11 commune.

De façon particulièrement préférentielle, les branches d'ancrage 10 sont toutes sensiblement de même forme, de mêmes dimensions et réparties de façon équidistante autour du premier tronçon 4.

Ainsi, les portions divergentes 10A des branches d'ancrage sont de préférence sensiblement contenues dans l'espace compris entre une enveloppe tronconique interne d'axe (XX') et d'angle au sommet égal à 10° et une enveloppe tronconique externe de même axe (XX') et d'angle au sommet égal à 40°.

Selon une caractéristique importante de l'invention, les branches d'ancrage 10 sont déployées par fabrication, tel que cela sera décrit plus en détails ci-après.

En d'autres termes, les branches d'ancrage 10 sont préformées dès l'origine, par construction, avant l'introduction et la mise en place de l'implant dans le ou les os 2,3.

En particulier les portions divergentes 10A sont pré-orientées par construction de manière à présenter intrinsèquement leur caractère divergent, et non à l'adopter après implantation.

L'implant 1 conforme à l'invention est donc de façon particulièrement préférentielle dépourvu de moyens de déploiement, propres ou rapportés, susceptibles de modifier activement sa configuration géométrique au cours de son implantation ou après cette dernière, et plus précisément susceptibles de déployer radialement les branches d'ancrage 10 au-delà de l'espace qu'elles occupent avant l'implantation, par exemple en forçant la déformation plastique sous contrainte de ces dernières.

L'implant 1 peut ainsi présenter avantageusement une structure particulièrement simple et intrinsèquement stable, dont le maintien est autonome et sensiblement invariant. L'utilisation d'un tel implant 1 limite avantageusement tout risque de fissuration d'un os ou de déplacement relatif incontrôlé d'un os 2, 3 par rapport à l'autre une fois l'implant introduit dans l'os.

Selon une autre caractéristique importante de l'invention, tel que cela est illustré sur les figures 1 à 4, les branches d'ancrage 10 présentent également une portion convergente 10B qui rabat sensiblement leur extrémité afin de faciliter l'introduction de l'implant 1 dans le premier os 2.

Ainsi, globalement, les jambes d'ancrage 10 présentent au moins une rupture de pente qui leur confère, vis-à-vis de l'extérieur, selon une direction transverse à l'axe d'extension (XX'), un profil globalement convexe (et plus particulièrement en V aplati).

Au sens de l'invention, le caractère convergent ou divergent des portions des branche d'ancrage 10A, 10B est de préférence continu sur toute la longueur de la portion considérée. Toutefois, il n'est pas exclu qu'une même portion de branche d'ancrage soit sujette localement à des variations d'éloignement de l'axe (XX'), telles que des ondulations, tout en conservant globalement son caractère divergent ou convergent.

De préférence, les branches d'ancrage 10 présentent au moins un coude 12, c'est-à-dire un changement de direction entre deux surfaces adjacentes ou deux segments consécutifs qui forment un angle saillant.

Avantageusement, le coude 12 marque une transition sensiblement continue entre la section divergente 10A et la section convergente 10B, et, de préférence, forme une portion globalement arrondie et d'épaisseur sensiblement constante et sensiblement égale à celles des portions adjacentes 10A, 10B.

Bien entendu, il est parfaitement envisageable au sens de l'invention que les branches d'ancrage 10 présentent plus de deux portions successives distinctes, et par exemple comprennent trois portions formant respectivement une «*grande*» portion divergente, suivie de deux «*petites*» portions convergentes, l'une intermédiaire et l'autre terminale, disposées en enfilade et rabattant chacune progressivement la branche vers le premier axe d'extension (XX').

Toutefois, lesdites branches d'ancrage 10 comprendront de préférence seulement deux portions successives, qui forment respectivement une portion divergente 10A s'étendant entre une extrémité captive du premier tronçon 4 et le coude 12, puis une portion convergente 10B qui s'étend entre le coude 12 et une extrémité libre opposée audit premier tronçon 4, laquelle peut avantageusement être totalement séparée des autres éléments constitutifs de l'implant. Un tel agencement confère avantageusement une certaine flexibilité structurelle et fonctionnelle au premier moyen de fixation 5, et favorise une légère déformation élastique fonctionnelle de la portion divergente 10A, lors de l'introduction de l'implant, laquelle assure une excellente prise dans l'os.

Ainsi, de préférence, tel que cela est illustré sur les figures 1 à 4, le coude 12 marque la transition entre la portion divergente 10A et une portion convergente 10B qui s'étend depuis ledit coude 12 jusqu'à une extrémité libre 14 de la branche d'ancrage 10, ladite extrémité libre 14 étant sensiblement rabattue vers le premier axe d'extension (XX').

Le coude 12 forme alors le sommet, ou «*point culminant*» de la branche d'ancrage 10, c'est-à-dire la partie la plus éloignée de l'axe d'extension (XX').

Selon une variante de réalisation préférentielle, l'angle de fermeture β selon lequel la portion convergente 10B est orientée par rapport au premier axe d'extension (XX') est sensiblement compris entre 0° et 30°.

Ainsi, par construction, l'angle du secteur angulaire délimité par le coude 12 et compris entre l'axe d'extension (XX') et ledit coude 12 est de préférence sensiblement compris entre 130° et 175°.

Il est remarquable que lorsque β = 0°, l'angle de divergence entre la portion divergente 10A et la portion convergente 10B est égal à la valeur de l'angle d'ouverture α, tandis que la portion convergente 10B s'étend sensiblement parallèlement à l'axe d'extension (XX').

Si β > 0°, la portion convergente 10B tend à rapprocher la partie terminale de la branche d'ancrage 10 du premier axe d'extension (XX').

Avantageusement, en recourbant ainsi la branche d'ancrage 10 conformément à l'invention, on peut former une rampe d'engagement qui facilite l'introduction de ladite branche d'ancrage 10, et plus généralement de l'implant 1, dans l'os 2. Plus globalement, les branches d'ancrage 10, et plus particulièrement leurs portions convergentes 10B, peuvent former dans leur ensemble une pointe sensiblement conique de pénétration dans l'os.

En outre, tel que cela est illustré sur la figure 4, une telle rampe peut avantageusement coopérer avec la paroi interne 151 d'un logement 15 creusé dans le premier os 2 de telle sorte que, lorsque la portion convergente 10B glisse le long de ladite paroi, cette dernière exerce une contrainte qui tend à fléchir la branche d'ancrage 10 en la rabattant vers le premier axe d'extension (XX'). De façon particulièrement avantageuse, les branches d'ancrage 10, sous l'effet de leur déformation élastique, et plus particulièrement de la déflexion de leur portion divergente 10A, peuvent alors exercer une pression contre la paroi interne 15l du logement 15, ledit logement 15 exerçant en retour, selon le principe de l'action-réaction, un effort de serrage sensiblement concentrique sur le moyen de fixation 5.

Les branches d'ancrage 10 conformes à l'invention sont donc de préférence déployées «*en parapluie*» de sorte à former sensiblement les génératrices d'une enveloppe bi-conique virtuelle dont l'axe coïncide sensiblement avec le premier axe d'extension (XX') et dont le plus grand diamètre de base D1 correspond à celui du cercle virtuel passant par les coudes 12.

En outre, la longueur de la portion divergente 10A représente de préférence environ 50 % à 90 % de la longueur totale d'une branche d'ancrage 10, tandis que la longueur de la portion convergente 10B représente de préférence environ 10 % à 50 % de la longueur totale de ladite branche d'ancrage 10.

En d'autres termes, le «*rabat d'introduction*» formé par la portion convergente 10B est sensiblement plus court que la «*patte de fixation*» formée par la portion divergente 10A correspondante.

Par ailleurs les branches d'ancrage 10 présenteront de préférence, sur la majeure partie sinon la totalité de leur longueur, des dimensions transverses, et notamment une épaisseur, sensiblement constantes (c'est-à-dire une section transverse sensiblement constante). Un tel agencement peut notamment être obtenu de manière très simple, tel que cela sera décrit plus en détails ci-après, en fabricant l'implant à partir d'un format brut tubulaire.

D'autre part, bien que les portions divergente 10A et convergente 10B, s'étendent de préférence de façon sensiblement rectiligne, il est également envisageable que celles-ci soient bombées selon leur direction longitudinale d'extension, et présentent une forme sensiblement incurvée, concave ou convexe.

Pour améliorer la fixation de l'implant 1 et notamment bloquer ce dernier selon le premier axe d'extension (XX') après son introduction dans le premier os 2, et plus particulièrement dans le logement 15, le sommet saillant du coude 12 est de préférence pourvu d'un organe anti-retour 16, tel qu'une barbe ou un ergot, agencé pour autoriser la progression du premier tronçon 4 dans le sens de la pénétration dans l'os et pour s'opposer à l'extraction hors du premier os 2 de la branche d'ancrage 10 correspondante. Plus précisément, ladite barbe est destinée à se ficher dans la paroi 151 du logement 15 de manière à ancrer la branche 10 dans le tissu osseux. Avantageusement, la pénétration de l'organe anti-retour 16 dans la paroi interne 151 est facilitée par la contrainte de plaquage du coude 12 contre la paroi interne 151 qu'exerce la branche d'ancrage 10, et plus particulièrement la portion divergente 10 de celle-ci, sous l'effet de sa déformation élastique.

Le maintien en translation, le long du premier axe d'extension (XX'), du premier tronçon 4 selon le sens de son introduction dans le premier os 2 peut par exemple être opéré soit par la mise en butée des extrémités libres 14 contre le fond du logement 15 lorsque ce dernier est borgne, soit par l'emprisonnement de matière osseuse dans l'espace compris entre les branches d'ancrage 10 lors de ladite introduction, ladite matière accumulée formant un coussin s'opposant à la migration de l'implant 1.

Au sens de l'invention, le second moyen de fixation 7 n'est pas limité à une forme particulière de réalisation et peut notamment faire appel à diverses solutions de fixation mécanique, tel que des vis, des broches ou encore des agrafes.

Toutefois, de façon préférentielle, le second moyen de fixation 7 reproduit sensiblement les solutions mises en oeuvre par le premier moyen de fixation 5, l'agencement et les fonctions dudit second moyen de fixation 7 se déduisant de ceux du premier moyen de fixation 5 mutatis mutandis.

Par commodité de description, les différents éléments constitutifs du second moyen de fixation 7 seront par conséquent numérotés en utilisant les références incrémentées d'une valeur 100 des éléments constitutifs analogues du premier moyen de fixation 5.

Plus particulièrement, le second moyen de fixation 7 comporte de préférence une pluralité de branches d'ancrage 110 solidaires du second tronçon 6 et agencées selon une disposition non coplanaire de sorte à créer une liaison d'encastrement entre l'implant 1 et le second os 3.

En outre, lesdites branches d'ancrage 110 sont déployées par fabrication de sorte à présenter une portion divergente qui s'ouvre vers l'extérieur par rapport au premier tronçon ainsi qu'une portion convergente rabattant sensiblement leur extrémité afin de faciliter l'introduction de l'implant 1 dans le second os 3.

De façon particulièrement préférentielle, l'implant 1 est sensiblement oblong et le second tronçon 6 est situé à l'extrémité dudit implant qui se trouve à l'opposé du premier tronçon 4.

De façon préférentielle, tel que cela est illustré sur les figures 1 à 4, les branches d'ancrage 110 associées au deuxième tronçon 6 sont également réparties en corolle autour de ce dernier et s'étendent au-delà de l'extrémité correspondante de l'implant 1, dans une direction sensiblement opposée à celle des branches d'ancrage 10 associées au premier tronçon 4.

Selon une variante de réalisation non représentée, la conformation du second moyen de fixation 7 est identique à celle du premier moyen de fixation 6.

En particulier, on peut réaliser l'implant 1 en rapportant bout à bout deux tronçons 4, 6 disposés tête-bêche, lesdits tronçons étant porteurs de moyens de fixation 5, 7 conformes à l'invention rigoureusement identiques, de sorte à former un implant 1 présentant un plan de symétrie miroir sensiblement normal au premier axe d'extension (XX').

Toutefois, il est parfaitement envisageable que la conformation du second moyen de fixation 7 diffère de celle du premier moyen de fixation 5 par sa géométrie, par ses dimensions, par le nombre des branches d'ancrage associées à chacun des tronçons respectifs, par le nombre et l'orientation des coudes, etc.

Ainsi, tel cela est représenté sur les figures 1, 3, 4, 6 et 7 la conformation du second moyen de fixation 7 diffère de préférence de celle du premier moyen de fixation 5 par ses dimensions, et plus spécifiquement par la longueur des branches d'ancrage 110, et/ou par l'angle d'ouverture de ces dernières par rapport à l'axe d'extension du second tronçon 6.

En particulier, si l'on considère que les branches d'ancrage 10, 110 sont déployées «*en parapluie*» de sorte à former sensiblement les génératrices d'une paire d'enveloppes bi-coniques virtuelles dont les axes respectifs coïncident sensiblement avec celui du tronçon correspondant et dont le plus grand diamètre de base («*diamètre hors-tout*»*)* correspond à celui du cercle virtuel passant par les coudes 12, 112, il est particulièrement avantageux que le diamètre hors-tout D1 au repos du premier moyen de fixation 5 situé sur l'extrémité distale de l'implant soit supérieur au plus grand diamètre hors-tout D2 au repos du second moyen de fixation 7 situé à l'extrémité proximale de l'implant 1.

En effet, lorsque l'on résèque les extrémités des phalanges, la phalange proximale, c'est-à-dire la plus proche de la cheville s'il s'agit d'une phalange de pied, présente une tête relativement étroite, tandis que la base de la phalange distale correspondante, celle qui se trouve la plus proche des orteils, est sensiblement plus large. Ainsi, un implant asymétrique permet avantageusement de tirer parti au mieux du volume d'ancrage disponible dans chaque phalange en maximisant la portée du moyen de fixation correspondant.

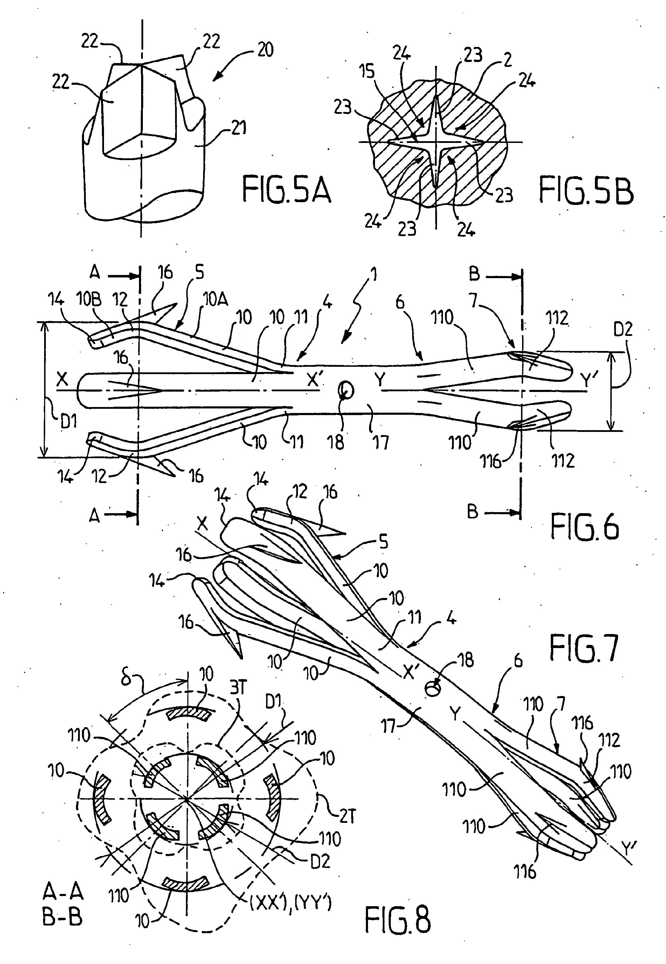

Selon des variantes de réalisation particulièrement préférentielles illustrées sur les figures 4 et 7, le premier tronçon 4 s'étendant sensiblement selon un premier axe d'extension (XX') et le second tronçon 6 s'étendant sensiblement selon un deuxième axe d'extension (YY'), l'implant 1 est cintré de telle sorte que ledit deuxième axe d'extension (YY') diverge du premier axe d'extension (XX').

De façon particulièrement préférentielle, l'angle de déviation η du second axe d'extension (YY') par rapport au premier axe d'extension (XX') est sensiblement compris entre 10° et 20°, et de préférence sensiblement égal à 15°. Un tel agencement permet en effet de reproduire idéalement l'angulation naturelle de la phalange proximale par rapport à la phalange distale et, plus précisément, l'angle formé par les axes médullaires desdites phalanges.

Plus généralement, il est envisageable de réaliser indifféremment des implants conformes à l'invention qui soient droits ou coudés de manière à adapter au cas par cas la géométrie dudit implant 1 à l'anatomie du patient et/ou de la zone traitée.

En outre, selon une caractéristique qui peut constituer une invention à part entière, indépendamment du pré-déploiement par construction ou de la forme des branches d'ancrage, la répartition des branches d'ancrage 10 associées au premier tronçon 4, considérée dans une section normale au premier axe d'extension (XX'), peut être décalée angulairement d'un angle δ par rapport à la répartition des branches d'ancrage 110 associées au second tronçon 6, considérée dans une section normale au second axe d'extension (YY').

Plus précisément, tel que cela est représenté sur les figures 6, 7, et 8, le premier moyen de fixation 5 peut comprendre un premier réseau de branches d'ancrage 10 tandis que le second moyen de fixation 7 comprend un second réseau de branches d'ancrage 110 qui comporte le même nombre de branches que le premier réseau (quatre en l'occurrence) agencées selon une géométrie sensiblement analogue à celle dudit premier réseau, lesdits premier et second réseaux de branches présentant entre eux un décalage angulaire δ, ou «*déphasage*», sensiblement égal à 45°.

En effet, les sections transversales 2T, 3T, (représentées en pointillés sur la figure 8) de deux phalanges successives présentent généralement une ou plusieurs directions d'extension privilégiées et non un rayon constant sous tous les azimuts, c'est-à-dire que lesdites sections transversales 2T, 3T ne sont généralement pas circulaires mais plutôt ovoïdes ou polylobées. L'implant conforme à l'invention permet avantageusement de reproduire le décalage naturel d'orientation existant entre lesdites directions d'extension privilégiées propres à chaque section transversale et ainsi de tirer parti dans chaque phalange des zones les plus fournies en matière osseuse pour améliorer la fixation, tant en étendue, donc en stabilité, qu'en résistance.

Bien entendu, la valeur du déphasage sera avantageusement choisie en fonction des os 2, 3 concernés par l'opération et plus précisément en fonction de la disposition de leurs sections transversales saignantes respectives.

De préférence, l'implant 1 conforme à l'invention est monobloc. Ainsi, le premier tronçon 4 et le second tronçon 6 sont de préférence venus de matière et fusionnent l'un avec l'autre pour former un tronc commun central 17 à partir duquel s'étendent, de part et d'autre, les branches d'ancrage 10, 110.

Bien entendu, l'implant 1 conforme à l'invention est réalisé dans un ou plusieurs matériaux biocompatibles, de préférence en titane. Il est par ailleurs envisageable, sans sortir du cadre de l'invention, de réaliser un tel implant dans un matériau biorésorbable, tel que du polylactide, de manière à ce que ledit implant soit assimilé par l'organisme une fois la fusion osseuse effectuée.

Bien entendu, on choisira un matériau suffisamment rigide pour que les branches d'ancrage puissent remplir leur rôle d'immobilisation et supporter l'introduction dans l'os alors qu'elles sont déjà déployées.

Selon une variante de réalisation particulièrement préférentielle, l'implant 1 est formé par un élément tubulaire dont la paroi est fendue à ses extrémités de telle sorte que lesdites extrémités sont scindées en une pluralité de languettes formant les branches d'ancrage 10, 110. Il est envisageable que l'élément tubulaire soit formé par un profilé de section quelconque, éventuellement ouverte (en U, en T, en L, carré, rond, etc:).

De préférence, lesdites extrémités sont fendues longitudinalement et évasées, c'est-à-dire que les languettes sont déployées radialement dans l'espace de manière à s'ouvrir vers l'extérieur par rapport à la section initiale de l'élément tubulaire, et plus particulièrement à former sensiblement une paire de cônes opposés par le sommet, tel que cela est illustré sur les figures 1 à 4, 6 et 7. Ainsi, le tronc 17 est de préférence longiligne et de largeur inférieure aux diamètres hors-tout D1, D2 des réseaux de branches d'ancrages 10, 110 qu'il sépare.

L'utilisation d'un élément tubulaire de base permet notamment, tel que cela sera décrit plus en détails ci-après, une fabrication simple et peu coûteuse dudit implant 1. Elle confère en outre à l'implant 1 sa légèreté tout en lui conservant une bonne rigidité en flexion et une bonne résistance mécanique à la torsion.

Par ailleurs, tel que cela est représenté sur les figures 6 et 7, l'implant 1 conforme à l'invention comprend de préférence un moyen de préhension 18 pourvu d'un détrompeur agencé pour renseigner l'utilisateur sur l'orientation dudit implant 1 lors de la saisie et de la manipulation de ce dernier.

En effet, l'existence d'un cintrage et/ou de différences géométriques ou dimensionnelles entre le premier moyen de fixation 5 et le second moyen de fixation 7 requiert un positionnement précis de l'implant 1 au regard du premier et du second os, selon une orientation particulière.

Le moyen de préhension 18 conforme à l'invention pourra notamment être réalisé à l'aide d'un perçage réalisé dans le tronc central 17 de l'implant situé entre les deux tronçons 4, 6, de telle sorte qu'un outil de forme conjuguée comportant un tenon destiné à venir coopérer de façon unique et reproductible avec ledit perçage permette au chirurgien de s'assurer de l'orientation directionnelle (sens d'introduction) et angulaire (position anatomique) de l'implant lors de la mise en place de ce dernier.

La présente invention peut également se rapporter à un instrument chirurgical 20 de préparation osseuse en tant que tel. Ledit instrument 20 est notamment destiné à la préparation d'un os 2 en vue d'y loger un implant 1 présentant au moins un premier tronçon 4 conçu pour être introduit dans ledit os et pourvu d'un premier moyen de fixation 5 agencé pour retenir ledit implant au sein dudit os.

En référence à la description qui précède, un instrument conforme à l'invention peut être employé indifféremment pour la préparation du premier et/ou du second os.

Selon l'invention, le premier moyen de fixation 5 comportant une pluralité de branches d'ancrage 10 solidaires dudit premier tronçon 4 et agencées selon une disposition non coplanaire de sorte à créer une liaison d'encastrement entre ledit implant 1 et ledit os 2, 3, l'instrument 20 comprend un poinçon 21 présentant une pluralité d'éléments saillants 22 agencés pour marquer simultanément dans l'os 2, 3, par percussion, une empreinte comprenant une pluralité de cavités 23 destinées à accueillir lesdites branches d'ancrage 10, l'agencement desdites cavités 23 étant sensiblement conjugué à celui desdites branches d'ancrage 10.

Bien entendu, ledit instrument 20 n'est limité ni à une forme ni à des dimensions particulières de réalisation et pourra aisément être adapté en fonction des différentes variantes de réalisation de l'implant 1 décrites précédemment.

Toutefois, le premier moyen de fixation 5 de l'implant 1 comportant de préférence quatre branches d'ancrage 10 sensiblement équidistantes, le poinçon 21 présentera une géométrie cruciforme, tel que cela est illustré sur la figure 5A, destinée à fendre l'os 2 selon quatre cavités 23 contiguës sensiblement distinctes.

Ainsi que cela est représenté sur la figure 5B, lesdites cavités 23 peuvent former les lobes d'un même logement 15, chacun de ces lobes étant destiné à accueillir une branche d'ancrage 10. Naturellement, le nombre, la disposition et les dimensions des cavités 23 peuvent être adaptés en fonction de l'agencement des branches d'ancrage 10, et notamment former une étoile.

Il est remarquable que le recours à un logement 15 comportant une pluralité de cavités 23 séparées l'une de l'autre par des éléments de cloison 24 permet avantageusement de renforcer le blocage en rotation du premier tronçon 4 par rapport au premier os 2 selon le premier axe d'extension (XX').

Bien entendu, la présente invention peut également concerner en tant que tel un kit chirurgical comprenant :
- d'une part un implant 1 conforme à l'invention, et plus particulièrement destiné à être interposé entre un premier os 2 et un second os 3 afin de maintenir lesdits premiers et second os sensiblement accolés l'un à l'autre en vue d'obtenir la fusion osseuse de ces derniers, ledit implant 1 présentant au moins un premier tronçon 4 conçu pour être introduit dans le premier os et pourvu d'un premier moyen de fixation 5 agencé pour retenir ledit implant 1 au sein du premier os 2, ainsi qu'un second tronçon 6 conçu pour être introduit dans le second os 3 et pourvu d'un second moyen de fixation 7 agencé pour retenir ledit implant 1 au sein du second os 3, ledit premier moyen de fixation 5 comportant une pluralité de branches d'ancrage 10 solidaires du premier tronçon 4 et agencées selon une disposition non coplanaire de sorte à pouvoir créer une liaison encastrement entre ledit implant 1 et ledit premier os 2,
- et d'autre part un instrument chirurgical 20 destiné à la préparation osseuse, ledit instrument comprenant un poinçon 21 qui présente une pluralité d'éléments saillants 22 agencés pour marquer simultanément dans l'os, par percussion, une empreinte comprenant une pluralité de cavités 23 destinées à accueillir lesdites branches d'ancrage 10, l'agencement desdites cavités 23 étant sensiblement conjugué à celui desdites branches d'ancrage 10.

L'utilisation d'un implant conforme à l'invention pour réaliser une arthrodèse inter-phalangienne va maintenant être décrite en détails, en référence préférentielle à un kit chirurgical conforme à l'invention.

Le praticien commence par réaliser la préparation du premier et du second os 2, 3, en réséquant les extrémités de ces derniers de manière à former des surfaces de contact saignantes de géométrie régulière, de préférence sensiblement planes.

Plus précisément, le praticien excise la base de la phalange distale (le premier os 2 sur la figure 2) ainsi que la tête de la phalange proximale (le second os 3 sur la figure 2).

Il ménage ensuite dans chaque extrémité ainsi mise à nue un logement destiné à accueillir l'implant 1.

Pour ce faire, il peut par exemple placer contre la surface saignante un instrument 20 formé d'une tige comportant à l'une de ses extrémités un poinçon 21 cruciforme et à l'autre extrémité une enclume, puis introduire en force les éléments saillants 22 dans le premier os 2 en venant percuter l'enclume à une ou plusieurs reprises à l'aide d'un marteau approprié.

La réalisation du logement 15 peut également comporter une étape de perçage au cours de laquelle on vient aléser l'os sensiblement selon son axe médullaire.

Bien entendu, selon la qualité du tissu osseux rencontré et la configuration de l'articulation à traiter, il est envisageable soit de poinçonner directement l'os, soit d'aléser un simple logement 15 circulaire au foret, soit de procéder d'abord au perçage d'un premier trou sensiblement cylindrique puis seulement ensuite de venir marquer au fond et/ou dans les parois dudit trou les cavités 23 à l'aide du poinçon cruciforme 21.

Le praticien saisit ensuite l'implant 1 à l'aide d'un instrument de préhension adapté, de type pince, en prenant soin de positionner ledit implant 1 convenablement par rapport à ladite pince à l'aide du moyen de préhension 18. Plus précisément, ladite pince peut comporter un canal au sein duquel fait saillie un tenon, l'implant étant alors positionné en plaçant le tronc central 17 tubulaire dans le canal de telle sorte que le tenon s'enclenche dans le trou de détrompage ménagé dans ledit tronc.

Une fois l'implant 1 assujetti à la pince, celui-ci est introduit en force dans le logement 15. Pour cela, le praticien vient appuyer les extrémités libres 14 des branches d'ancrage 10 contre la paroi interne 15l, au niveau du col du logement 15, puis fait progressivement pénétrer par compression et/ou par percussion le premier tronçon 4 à l'intérieur dudit logement 15.

Plus spécifiquement, dans le cas d'un implant 1 comprenant quatre branches d'ancrage 10 équidistantes tel que cela est représenté sur les figures 1, 3, 4, 6 et 7, le praticien vient positionner chacune des branches d'ancrage 10 en regard de l'une des cavités 23 de l'empreinte cruciforme.

Avantageusement, l'implant 1 est introduit tel quel, sous sa forme pré-déployée par construction, les branches d'ancrage 10 déjà écartées l'une de l'autre, tandis que le premier axe d'extension (XX') se présentant de façon sensiblement parallèle, voire sensiblement coaxiale, avec l'axe médullaire du premier os 2.

De préférence, le diamètre du logement 15 est sensiblement inférieur au diamètre hors-tout D1 de déploiement au repos des branches d'ancrage 10, si bien que, lors de l'introduction du premier tronçon 4 dans le logement 15, la portion convergente 10B desdites branches vient coopérer avec la paroi interne 15l à la manière d'une rampe et fait fléchir progressivement lesdites branches 10 en les rabattant vers le premier axe d'extension (XX'), c'est-à-dire réduit l'angle d'ouverture α, au moins jusqu'à ce que ledit premier tronçon soit enfoncé à hauteur des coudes 12.

Une fois que les coudes 12 ont franchi le col du logement 15, ceux-ci continuent à glisser avec frottement le long de la paroi interne 151 dudit logement, au fur et à mesure que le premier tronçon 4 s'enfonce dans ce dernier.

En contraignant ainsi les branches à se contracter pour que les coudes se positionnent sur un diamètre sensiblement inférieur à celui qu'ils occupent par défaut, une contrainte élastique de déformation apparaît entre le premier moyen de fixation 5 et le logement 15, ce qui contribue à fournir un serrage concentrique de premier tronçon 4, et par conséquent de l'implant 1, au sein dudit logement 15.

En outre, les organes anti-retour 16 coopèrent avec la paroi interne 151 en pénétrant dans le tissu osseux périphérique du logement 15 au fur et à mesure de la progression du premier tronçon dans ledit logement 15, assurant ainsi à tout moment une prise stable de l'implant par encliquetage.

Le praticien cesse d'enfoncer le premier tronçon 4 dans le premier os 2 lorsque les extrémités libres 14 des branches d'ancrage 10 atteignent le fond du logement 15 et/ou lorsque le plan de jonction entre le premier tronçon 4 et le second tronçon 6 affleure sensiblement à la surface saignante dudit os.

Il est remarquable que la réalisation d'un logement 15 peut s'avérer facultative, les branches d'ancrage 10 e/ou le premier tronçon 4 dans son ensemble étant alors enfoncés directement dans la matière du premier os 2, un peu à la manière de clous, à partir de la surface saignante. Les extrémités libres 14 peuvent à cet effet être biseautées de manière à faciliter leur pénétration dans les tissus.

Une fois le premier tronçon 4 fermement ancré dans le premier os 2, le praticien peut introduire, de manière sensiblement analogue, le second tronçon 6 dans le second os 3.

En particulier, la préparation du deuxième os 3 peut être sensiblement similaire à celle du premier os 2.

Bien entendu, la méthode d'implantation de l'implant 1 conforme à l'invention peut faire l'objet de variantes, notamment en fonction de l'ordre dans lequel les premier et second tronçons sont introduits respectivement dans le premier et le second os. En variante, il est même envisageable d'introduire simultanément le premier et le second tronçon dans le premier et le second os respectivement en positionnant les branches d'ancrage 10, 110 de chaque tronçon au contact du bord du logement 15, 115 ménagé dans l'os correspondant 2, 3, puis en rapprochant le premier os 2 du second os 3 de manière à faire pénétrer simultanément lesdits tronçons dans lesdits logements.

Il est remarquable qu'une fois le premier et le second tronçon introduits dans leur os respectifs, le premier et le second os se trouvent immédiatement et automatiquement arrimés l'un à l'autre par l'implant 1, sans qu 'il soit nécessaire de modifier la conformation de ce dernier. Ledit implant 1 réalise avantageusement entre eux une jonction stable et rigide, laquelle supprime notamment toute possibilité de séparation spontanée ou de mouvement relatif desdits os, notamment en rotation autour de leurs axes médullaires respectifs et en flexion au niveau de l'ancienne interface articulaire.

Par ailleurs, l'implant 1 conforme à l'invention permet avantageusement de réaliser une implantation médullaire strictement interne, tel que cela est illustré sur la figure 2, ledit implant se trouvant in fine noyé totalement dans la masse osseuse, et plus précisément chacun des tronçons se trouvant inséré en intégralité dans la diaphyse et/ou l'apophyse de l'os correspondant. Avantageusement, aucune partie dudit implant 1 n'est donc apparente une fois le premier et le second os réuni, ni a fortiori affleurante ou saillante par rapport aux tissus corticaux desdits os. Il en résulte une utilisation confortable et sûre dudit implant, non traumatisante pour les tissus environnants.

En outre, l'implant 1 conforme à l'invention et la méthode d'implantation associée minimisent le travail de préparation des os à fixer, en rendant notamment superflu le perçage de longs canaux d'accès, notamment traversant l'un et/ou l'autre des os de part en part, et/ou les usinages complexes dans des parties fragiles desdits os.

La présente invention se rapporte enfin à un procédé de fabrication d'un implant médical pourvu d'un moyen de fixation 5, 7 agencé pour retenir ledit implant 1 au sein d'un os 2, 3.

Selon l'invention, un tel procédé de fabrication comprend une étape (a) de réalisation dudit moyen de fixation 5, 7 au cours de laquelle on entaille une pièce oblongue de manière à diviser une fraction de cette dernière en une pluralité de branches d'ancrage 10, 110 non coplanaires et solidaires de ladite pièce oblongue.

Bien qu'elle puisse être formée par un rondin ou une autre pièce massive, l'étape (a) impliquant alors une taille dans la masse, la pièce oblongue est de préférence formée par un élément creux, tel qu'un profilé, et de façon particulièrement préférentielle par un tube de section circulaire.

L'étape (a) de réalisation du moyen de fixation comporte alors de préférence une sous-étape (a1) de découpe au cours de laquelle on réalise à partir de l'une des extrémités dudit élément creux une ou plusieurs découpes traversant la paroi de ce dernier, de manière à scinder l'extrémité dudit élément creux en une pluralité de languettes.

De façon particulièrement préférentielle, les découpes ainsi effectuées sont agencées de manière à fendre la pièce oblongue longitudinalement selon son axe principal d'extension.

La découpe peut avantageusement être réalisée au laser, ce procédé garantissant une grande précision pour la réalisation d'implants 1 de petites dimensions.

En outre, selon une caractéristique importante de l'invention, ladite étape (a) de réalisation du moyen de fixation comporte une sous-étape (a2) de déploiement au cours de laquelle on plie lesdites languettes vers l'extérieur de sorte à former, avec lesdites languettes, des branches d'ancrage 10, 110 qui divergent selon une répartition sensiblement tronconique.

En d'autres termes, au cours de la sous-étape (a2) de déploiement de portion divergente 10A, on plie les branches d'ancrage 10 vers l'extérieur de sorte à les faire diverger selon une répartition sensiblement tronconique.

De façon imagée, le procédé de fabrication conforme à l'invention consiste de préférence sensiblement à «*éplucher*» les parois de l'élément creux, à les déformer plastiquement pour éclater l'une et/ou l'autre des extrémités dudit élément creux en corolle.

L'étape (a) peut en outre comporter une sous-étape de pliage (a3) au cours de laquelle on forme les coudes 12, 112 en rabattant les extrémités libres des languettes vers l'axe d'extension de la pièce oblongue.

La sous-étape (a3) constitue donc une sous-étape de formation de portion convergente 10B au cours de laquelle on rabat par pliage les extrémités libres desdites branches d'ancrage vers l'axe d'extension de la pièce oblongue.

Avantageusement, il est possible de réaliser, préalablement à la sous-étape de pliage (a3), des traits de découpes en V au niveau où doivent être formés les coudes 12, 112, de telle manière que le pliage mis en oeuvre pour marquer lesdits coudes fasse spontanément saillir les organes anti-retour 16, 116, en l'occurrence au sommet desdits coudes, au niveau du pli.

Chaque organe anti-retour est ainsi mis en forme au cours de la même opération que le coude correspondant.

Il est remarquable que le recours à un élément creux permet avantageusement de limiter la masse de matière mise en oeuvre, de réduire l'énergie nécessaire à l'usinage, d'alléger l'implant sans compromettre sa tenue aux sollicitations mécaniques et enfin de modérer le prix de revient dudit implant. Le tube de section circulaire constitue quant à lui un format brut privilégié dans la mesure où il s'agit d'un format commun à de nombreux matériaux biocompatibles et largement disponibles en diverses tailles sur le marché.

L'étape (a), et plus précisément les sous-étapes (a1) et (a2) décrites ci-dessus peuvent naturellement être répétées, séquentiellement ou simultanément, à chacune des extrémités de la pièce oblongue de manière à façonner un implant 1 conforme aux variantes de réalisation illustrées sur les figures 1, 3, 4, 6 et 7.

Toutefois, le procédé de fabrication conforme à l'invention n'est nullement limité à ce type d'implant et peut notamment être mis en oeuvre pour réaliser des implants présentant un seul tronçon (ou une seule face) de fixation, tels que des agrafes ou autres punaises destinées à maintenir par pincement ou compression un tissu quelconque (os, muscle, tendon...) contre un os et présentant, à l'opposé du tronçon de fixation, une face aux contours atraumatiques. De tels implants peuvent notamment être fixés dans la zone corticale d'un os, par exemple en étant rapportés contre la surface externe dudit os selon un abord radial.

Le procédé peut également comporter une étape (b) de cintrage au cours de laquelle on déforme plastiquement la pièce oblongue pour conférer à l'implant 1, une forme ergonomique qui reproduise sensiblement la configuration anatomique naturelle de la zone d'implantation dudit implant. Ladite étape de cintrage (b) peut intervenir avant ou après l'étape (a) de réalisation du ou des moyens de fixation.

A titre d'exemple non limitatif, dans le cas de la réalisation d'un implant 1 inter-phalangien destiné à la chirurgie du pied, on pourra appliquer le procédé de fabrication, et plus précisément les sous-étapes (a1) de découpe, (a2) de déploiement et (a3) de pliage, à un tube en titane de diamètre intérieur égal à 2,2 mm et d'épaisseur de paroi égale à 0,5 mm de manière à réaliser un implant d'une longueur hors-tout de l'ordre de 15 mm à 30 mm, de préférence de 17 mm à 25 mm, et de diamètre hors-tout compris entre 2,7 mm et 4,5 mm.

Ainsi, l'implant 1 conforme à l'invention présente une excellente assise dans les os, laquelle est due non seulement à la multiplicité des points d'ancrage mais également à la géométrie de ses moyens de fixation qui confèrent aux liaisons encastrement réalisées une tenue ferme et rigide, quelle que soit la direction de sollicitation mécanique de ladite liaison.

En outre, un tel implant permet de réaliser la fixation d'un premier os à un second os en minimisant l'importance et la durée du geste chirurgical, notamment de la préparation des os.

En particulier, la géométrie de l'implant 1 facilite son introduction et sa progression dans l'os, tout en assurant une prise mécanique immédiate et «*automatique*» de l'implant dans ledit os dès son introduction, sans requérir de manipulation ultérieure.

Par ailleurs, la simplicité de réalisation d'un tel implant permet avantageusement de le produire à un coût particulièrement compétitif.

Enfin, un tel implant pouvant être adapté dans sa géométrie comme dans ses dimensions pour des usages variés dans différentes configurations de zones de fixation, et notamment d'articulations, sa polyvalence et son ergonomie garantissent un excellent confort d'utilisation pour le patient.

## Revendications

1. Implant médical (1) destiné à être interposé entre un premier os (2) et un second os (3) afin de maintenir lesdits premier et second os (2, 3) sensiblement accolés l'un à l'autre en vue d'obtenir la fusion osseuse de ces derniers, ledit implant (1) présentant au moins un premier tronçon (4) conçu pour être introduit dans le premier os (2) et pourvu d'un premier moyen de fixation (5) agencé pour retenir ledit implant (1) au sein du premier os (2), ainsi qu'un second tronçon (6) conçu pour être introduit dans le second os (3) et pourvu d'un second moyen de fixation (7) agencé pour retenir ledit implant au sein du second os (3), le premier moyen de fixation (5) comportant une pluralité de branches d'ancrage (10) non coplanaires et solidaires du premier tronçon (4) qui sont destinées à créer une liaison d'encastrement entre l'implant (1) et le premier os (2), ledit implant (1) étant **caractérisé en ce que** lesdites branches d'ancrage (10) sont déployées par fabrication de sorte à pouvoir être introduites dans l'os alors qu'elles sont déjà déployées, lesdites branches d'ancrage présentant, avant la mise en place et lors de l'introduction de l'implant (1) dans les os, une portion divergente (10A) qui s'ouvre vers l'extérieur par rapport au premier tronçon ainsi qu'une portion convergente (10B) rabattant sensiblement leur extrémité afin de faciliter l'introduction dudit implant (1) sous sa forme déployée dans ledit premier os (2).

2. Implant selon la revendication 1 **caractérisé en ce que** les branches d'ancrage (10) présentent au moins un pli formant une coude (12) qui marque la transition entre la portion divergente (10A) et la portion convergente (10B).

3. Implant selon la revendication 2 **caractérisé en ce que** le sommet saillant du coude (12) est pourvu d'un organe anti-retour (16), tel qu'une barbe, agencé pour s'opposer à l'extraction hors du premier os (2) de la branche d'ancrage (10) correspondante.

4. Implant selon l'une des revendications 2 ou 3 **caractérisé en ce que** la portion convergente (10B) s'étend depuis le coude (12) jusqu'à une extrémité libre (14) de la branche d'ancrage (10) considérée.

5. Implant selon l'une des revendications précédentes **caractérisé en ce que** la longueur de la portion divergente (10A) représente environ 50 % à 90 % de la longueur totale de la branche d'ancrage (10) considérée.

6. Implant selon l'une des revendications précédentes **caractérisé en ce que**, le premier tronçon (4) s'étendant selon un premier axe d'extension (XX'), l'angle d'ouverture (α) de la portion divergente (10A) par rapport audit premier axe d'extension (XX') est sensiblement compris entre 5° et 20°.

7. Implant selon l'une des revendications précédentes **caractérisé en ce que**, le premier tronçon (4) s'étendant selon un premier axe d'extension (XX'), l'angle de fermeture (β) selon lequel la portion convergente (10B) est orientée par rapport audit premier axe d'extension (XX') est sensiblement compris entre 0° et 30°.

8. Implant selon l'une des revendications précédentes **caractérisé en ce que** les branches d'ancrage (10) sont réparties en corolle autour du premier tronçon (4).

9. Implant selon l'une des revendications précédentes **caractérisé en ce que** le premier tronçon (4) est situé à une extrémité de l'implant (1) et **en ce que** les branches d'ancrage (10) s'étendent à partir dudit premier tronçon (4) et sensiblement au-delà de ladite extrémité.

10. Implant selon l'une des revendications précédentes **caractérisé en ce que** les branches d'ancrage (10) sont venues de matière avec le premier tronçon (4).

11. Implant selon l'une des revendications précédentes **caractérisé en ce que** le premier moyen de fixation (5) comporte au moins trois, et de préférence quatre, branches d'ancrage (10).

12. Implant selon l'une des revendications précédentes **caractérisé en ce que** le second moyen de fixation (7) comporte une pluralité de branches d'ancrage (110) solidaires du second tronçon (6) et agencées selon une disposition non coplanaire de sorte à créer une liaison d'encastrement entre l'implant (1) et le second os (3) et **en ce que** lesdites branches d'ancrage (110) sont déployées par fabrication de sorte à présenter une portion divergente qui s'ouvre vers l'extérieur par rapport au premier tronçon ainsi qu'une portion convergente rabattant sensiblement leur extrémité afin de faciliter l'introduction dudit implant (1) dans ledit second os (3).

13. Implant selon la revendication 12 **caractérisé en ce que** la conformation du second moyen de fixation (7) diffère de celle du premier moyen de fixation (5) par ses dimensions.

14. Implant selon l'une des revendications 12 ou 13 **caractérisé en ce que**, le premier et le second tronçon (4, 6) s'étendant sensiblement selon un premier axe d'extension (XX') et selon un deuxième axe d'extension (YY') respectivement, la répartition des branches d'ancrage (10) associées au premier tronçon (4), considérée dans une section normale au premier axe d'extension (XX'), est décalée angulairement par rapport à la répartition des branches d'ancrage (110) associées au second tronçon (6), considérée dans une section normale au second axe d'extension (YY').

15. Implant selon la revendication 14 **caractérisé en ce que** le décalage angulaire (δ) entre le réseau des premières branches d'ancrage (10) et le réseau des secondes branches d'ancrage (110) est sensiblement égal à 45°.

16. Implant selon la revendication 12 **caractérisé en ce que** la conformation du second moyen de fixation (7) est identique à celle du premier moyen de fixation (5).

17. Implant selon l'une des revendications précédentes **caractérisé en ce qu'**il est monobloc.

18. Implant selon l'une des revendications précédentes **caractérisé en ce qu'**il est formé par un élément tubulaire dont la paroi est fendue à ses extrémités de telle sorte que lesdites extrémités dudit tube sont scindées en une pluralité languettes formant les branches d'ancrage (10).

19. Implant selon l'une des revendications précédentes **caractérisé en ce que**, le premier et le second tronçon (4, 6) s'étendant sensiblement selon un premier axe d'extension (XX') et selon un deuxième axe d'extension (YY') respectivement, ledit implant (1) est cintré de sorte que l'angle de déviation (η) du second axe d'extension (YY') par rapport au premier axe d'extension (XX') est sensiblement compris entre 10° et 20°, et de préférence sensiblement égal à 15°.

20. Implant selon l'une des revendications précédentes **caractérisé en ce qu'**il comprend un moyen de préhension (18) pourvu d'un détrompeur agencé pour renseigner l'utilisateur sur l'orientation dudit implant lors de la saisie et de la manipulation de ce dernier.

21. Implant selon l'une des revendications précédentes **caractérisé en ce qu'**il constitue un implant d'arthrodèse inter-phalangienne.

22. Kit chirurgical **caractérisé en ce qu'**il comprend :
- d'une part un implant (1) selon l'une des revendications 1 à 21,
- et d'autre part un instrument chirurgical (20) destiné à la préparation osseuse, ledit instrument comprenant un poinçon (21) qui présente une pluralité d'éléments saillants (22) agencés pour marquer simultanément dans l'os, par percussion, une empreinte comprenant une pluralité de cavités (23) destinées à accueillir lesdites branches d'ancrage (10), l'agencement desdites cavités (23) étant sensiblement conjugué à celui desdites branches d'ancrage (10).

23. Procédé de fabrication d'un implant médical (1) conforme à l'une des revendications 1 à 21, ledit procédé comprenant une étape (a) de réalisation du premier moyen de fixation (5) au cours de laquelle on entaille une pièce oblongue de manière à diviser une fraction de cette dernière en une pluralité de branches d'ancrage (10) non coplanaires et solidaires de ladite pièce oblongue, ledit procédé étant **caractérisé en ce que** l'étape (a) de réalisation du premier moyen de fixation comporte une sous-étape (a2) de déploiement de portion divergente (10A) au cours de laquelle on plie lesdites branches d'ancrage (10) vers l'extérieur de sorte à les faire diverger selon une répartition sensiblement tronconique, ainsi qu'une sous-étape (a3) de formation de portion convergente (10B) au cours de laquelle on rabat par pliage les extrémités libres desdites branches d'ancrage vers l'axe d'extension de la pièce oblongue.

24. Procédé de fabrication selon la revendication 23 **caractérisé en ce que** la sous-étape (a3) de formation de portion convergente permet de former un coude (12) par pliage entre la portion divergente (10A) et la portion convergente (10B), et **en ce que** ladite sous-étape (a3) de formation de portion convergente est précédée par la réalisation de traits de découpe de telle sorte que le marquage dudit coude (12) par pliage fasse spontanément saillir au sommet de ce dernier un organe anti-retour (16), tel qu'une barbe.

## Claims

1. Medical implant (1) intended to be interposed between a first bone (2) and a second bone (3) in order to support said first and second bones (2, 3) substantially joined one to the other, so as to obtain bony fusion of the latter, said implant (1) having at least one first section (4) designed to be introduced into the first bone (2) and provided with a first means (5) of fixing arranged to hold said implant (1) in the first bone (2), as well as a second section (6) designed to be introduced into the second bone (3) and provided with a second means (7) of fixing arranged to hold said implant in the second bone (3), the first means (5) of fixing incorporating a plurality of non-coplanar anchoring branches (10) connected to the first section (4) which are intended to create a built-in link between the implant (1) and the first bone (2), said implant (1) being **characterised in that** said anchoring branches (10) are deployed during manufacture so that they can be introduced into the bone as they are already deployed, said anchoring branches having, before installation and during introduction of the implant (1) in the bones, a divergent portion (10A) which opens outwards relative to the first section (4) as well as a convergent portion (10B) turning down their ends substantially in order to facilitate the introduction of said implant (1) into said first bone (2) while being in its deployed form.

2. Implant according to claim 1, **characterised in that** the anchoring branches (10) have at least one fold forming a bend (12) which marks the transition between the divergent portion (10A) and the convergent portion (10B).

3. Implant according to claim 2, **characterised in that** the prominent apex of the bend (12) is provided with an anti-return device (16), such as a barb, arranged so as to oppose the extraction from the first bone (2) of the corresponding anchoring branch (10).

4. Implant according to one of claims 2 or 3, **characterised in that** the convergent portion (10B) extends from the bend (12) as far as a free end (14) of the anchoring branch (10) in question.

5. Implant according to one of the preceding claims, **characterised in that** the length of the divergent portion (10A) accounts for approximately 50% to 90% of the total length of the anchoring branch (10) in question.

6. Implant according to one of the preceding claims, **characterised in that** the first section (4) being extended according to a first axis of extension (XX'), the angle of opening (α) of the divergent portion (10A) relative to said first axis of extension (XX') is substantially between 5° and 20° inclusive.

7. Implant according to one of the preceding claims, **characterised in that** the first section (4) being extended according to a first axis of extension (XX'), the angle of closing (β) according to which the convergent portion (10B) is oriented relative to said first axis of extension (XX') is substantially between 0° and 30° inclusive.

8. Implant according to one of the preceding claims, **characterised in that** the anchoring branches (10) are distributed as a corolla about the first section (4).

9. Implant according to one of the preceding claims, **characterised in that** the first section (4) is located at one end of the implant (1) and **in that** the anchoring branches (10) extend from said first section (4) and substantially beyond said end.

10. Implant according to one of the preceding claims, **characterised in that** the anchoring branches (10) are of one piece with the first section (4).

11. Implant according to one of the preceding claims, **characterised in that** the first means (5) of fixing incorporate at least three and, more preferably, four anchoring branches (10).

12. Implant according to one of the preceding claims, **characterised in that** the second means (7) of fixing incorporates a plurality of anchoring branches (110) integral with the second section (6) and arranged according to a non-coplanar layout so as to create a built-in link between the implant (1) and the second bone (3) and **in that** said anchoring branches (110) are deployed during manufacture so as to have a divergent portion which opens outwards relative to the first section as well as a convergent portion turning down their ends substantially in order to facilitate the introduction of said implant (1) in said second bone (3).

13. Implant according to claim 12, **characterised in that** the conformation of the second means (7) of fixing differs from that of the first means (5) of fixing by its dimensions.

14. Implant according to one of claims 12 or 13, **characterised in that** the first and second sections (4, 6) extending substantially according to a first axis of extension (XX') and according to a second axis of extension (YY') respectively, the distribution of the anchoring branches (10) associated with the first section (4), considered in a section normal to the first axis of extension (XX'), is offset at an angle relative to the distribution of the anchoring branches (110) associated with the second section (6), considered in a section normal to the second axis of extension (YY').

15. Implant according to claim 14, **characterised in that** the angular offset (δ) between the network of the first anchoring branches (10) and the network of the second anchoring branches (110) is substantially equal to 45°.

16. Implant according to claim 12, **characterised in that** the conformation of the second means (7) of fixing is identical to that of the first means (5) of fixing.

17. Implant according to one of the preceding claims, **characterised in that** it is monobloc.

18. Implant according to one of the preceding claims, **characterised in that** it is formed by a tubular element in which the wall is split at its ends such that said ends of said tube are divided into a plurality of tongues forming the anchoring branches (10).

19. Implant according to one of the preceding claims, **characterised in that** the first and second sections (4, 6) being extended substantially according to a first axis of extension (XX') and according to a second axis of extension (YY') respectively, said implant (1) is bent such that the angle of deviation (η) of the second axis of extension (YY') relative to the first axis of extension (XX') is substantially between 10° and 20°, and more preferably substantially equal to 15°.

20. Implant according to one of the preceding claims, **characterised in that** it comprises a means of prehension (18) provided with a means of foolproofing arranged so as to inform the user of the orientation of said implant at the time of gripping and handling the latter.

21. Implant according to one of the preceding claims, **characterised in that** it is an inter-phalangeal implant.

22. Surgical kit, **characterised in that** it comprises:
- first an implant (1) according to one of claims 1 to 21,
- and second a surgical instrument (20) intended for bony preparation, said instrument comprising a punch (21) which has a plurality of prominent elements (22) arranged so as to mark simultaneously in the bone, by percussion, an imprint comprising a plurality of cavities (23) intended to accommodate the anchoring branches (10) of the implant, the arrangement of said cavities (23) being substantially conjugate to that of said anchoring branches (10).

23. Method for manufacturing a medical implant (1) as in one of claims 1 to 21, said method comprising a step (a) for producing the first means (5) of fixing during which an oblong part is cut so as to divide a fraction of the latter into a plurality of said non-coplanar anchoring branches (10) connected to said oblong part, said method being **characterised in that** the step (a) for producing the first means of fixing includes a sub-step (a2) for deploying the divergent portion (10A) during which said anchoring branches (10) are folded outwards so as to cause them to diverge according to a substantially tapered distribution, as well as a sub-step (a3) forming a convergent portion (10B) during which the free ends of said anchoring branches are turned down by folding towards the axis of extension of the oblong part.

24. Method of manufacture according to claim 23, **characterised in that** the sub-step (a3) forming the convergent portion makes possible to form a bend (12) by folding between the divergent portion (10A) and the convergent portion (10B) and **in that** said sub-step (a3) forming the convergent portion is preceded by making cut lines such that the marking of said bend (12) by folding causes an anti-return device (16) such as a barb, to protrude at the apex of said bend.

## Patentansprüche

1. Medizinisches Implantat (1), das dazu bestimmt ist, zwischen einen ersten Knochen (2) und einen zweiten Knochen (3) eingesetzt zu werden, um den ersten und zweiten Knochen (2, 3) im Wesentlichen miteinander verbunden zu halten, um die Knochenfusion letzterer zu erhalten, wobei das Implantat (1) mindestens ein erstes Teilstück (4) aufweist, das konzipiert ist, um in den ersten Knochen (2) eingeführt zu werden und mit einem ersten Befestigungsmittel (5) versehen ist, das angeordnet ist, um das Implantat (1) im ersten Knochen (2) zu halten, sowie ein zweites Teilstück (6), das konzipiert ist, um in den zweiten Knochen (3) eingeführt zu werden und mit einem zweiten Befestigungsmittel (7) ausgestattet ist, das angeordnet ist, um das Implantat im zweiten Knochen (3) zu halten, wobei das erste Befestigungsmittel (5) eine Vielzahl von nicht-koplanaren Verankerungsarmen (10) umfasst, die mit dem ersten Abschnitt (4) eine Einheit bilden, die dazu bestimmt sind, eine einbettende Verbindung zwischen dem Implantat (1) und dem ersten Knochen (2) zu erzeugen, wobei das Implantat (1) **dadurch gekennzeichnet ist, dass** die Verankerungsarme (10) durch Herstellung derart gespreizt sind, dass sie in den Knochen eingeführt werden können, wenn sie bereits gespreizt sind, wobei die Verankerungsarme vor dem Einbringen und während des Einführens des Implantats (1) in die Knochen einen divergierenden Abschnitt (10A), der sich, bezogen auf das erste Teilstück, nach außen öffnet, sowie einen konvergierenden Abschnitt (10B) aufweisen, dessen Ende im Wesentlichen umgebogen ist, um das Einführen des Implantats (1) in dessen gespreizter Form in den ersten Knochen (2) zu erleichtern.

2. Implantat nach Anspruch 1, **dadurch gekennzeichnet, dass** die Verankerungsarme (10) mindestens einen Knick aufweisen, der eine Krümmung (12) bildet, der den Übergang zwischen dem divergierenden Abschnitt (10A) und dem konvergierenden Abschnitt (10B) markiert.

3. Implantat nach Anspruch 2, **dadurch gekennzeichnet, dass** die hervorstehende Spitze der Krümmung (12) mit einer auszugsperrenden Vorrichtung (16), wie etwa einem Widerhaken versehen ist, der angeordnet ist, um der Extraktion des entsprechenden Verankerungsarms (10) aus dem ersten Knochen (2) entgegenzuwirken.

4. Implantat nach einem der Ansprüche 2 oder 3, **dadurch gekennzeichnet, dass** sich der konvergierende Abschnitt (10B) von der Krümmung (12) bis zu einem freien Ende (14) des betrachteten Verankerungsarms (10) erstreckt.

5. Implantat nach einem der vorhergehenden Ansprüche, **dadurch gekennzeichnet, dass** die Länge des divergierenden Abschnitts (10A) etwa 50 % bis 90 % der Gesamtlänge des betrachteten Verankerungsarms (10) darstellt.

6. Implantat nach einem der vorhergehenden Ansprüche, **dadurch gekennzeichnet, dass** sich das erste Teilstück (4) gemäß einer ersten Ausdehnungsachse (XX') ausdehnt, wobei der Öffnungswinkel (α) des divergierenden Abschnitts (10A) bezogen auf die erste Ausdehnungsachse (XX') im Wesentlichen im Bereich zwischen 5 ° et 20 ° liegt.

7. Implantat nach einem der vorhergehenden Ansprüche, **dadurch gekennzeichnet, dass** sich das erste Teilstück (4) gemäß einer ersten Ausdehnungsachse (XX') ausdehnt, wobei der Schließwinkel (β) der gemäß dem konvergierenden Abschnitt (10B) bezogen auf die erste Ausdehnungsachse (XX') ausgerichtet ist, im Wesentlichen im Bereich zwischen 0 ° et 30 ° liegt.

8. Implantat nach einem der vorhergehenden Ansprüche, **dadurch gekennzeichnet, dass** die Verankerungsarme (10) kronenartig um das erste Teilstück (4) herum verteilt sind.

9. Implantat nach einem der vorhergehenden Ansprüche, **dadurch gekennzeichnet, dass** das erste Teilstück (4) an einem Ende des Implantats (1) liegt, und **dadurch**, dass die Verankerungsarme (10) sich ausgehend von dem ersten Teilstück (4) und im Wesentlichen über das Ende hinaus ausdehnen.

10. Implantat nach einem der vorhergehenden Ansprüche, **dadurch gekennzeichnet, dass** die Verankerungsarme (10) einteilig mit dem ersten Teilstück (4) sind.

11. Implantat nach einem der vorhergehenden Ansprüche, **dadurch gekennzeichnet, dass** das erste Befestigungsmittel (5) mindestens drei, und bevorzugt vier Verankerungsarme (10) umfasst.

12. Implantat nach einem der vorhergehenden Ansprüche, **dadurch gekennzeichnet, dass** das zweite Befestigungsmittel (7) eine Vielzahl von Verankerungsarmen (110) umfasst, die mit dem zweiten Teilstück (6) eine Einheit bilden und gemäß einer nicht koplanaren Anordnung angeordnet sind, um eine einbettende Verbindung zwischen dem Implantat (1) und dem zweiten Knochen (3) zu erzeugen, und **dadurch**, dass die Verankerungsarme (110) durch Herstellung so gespreizt sind, dass sie einen divergierenden Abschnitt, der sich, bezogen auf das erste Teilstück, nach außen öffnet, sowie einen konvergierenden Abschnitt aufweisen, dessen Ende im wesentlichen umgebogen ist, um das Einführen des Implantats (1) in den zweiten Knochen (3) zu erleichtern.

13. Implantat nach Anspruch 12, **dadurch gekennzeichnet, dass** sich die Gestalt des zweiten Befestigungsmittels (7) von der des ersten Befestigungsmittels (5) durch ihre Abmessungen unterscheidet.

14. Implantat nach einem der Ansprüche 12 oder 13, **dadurch gekennzeichnet, dass** sich das erste und das zweite Teilstück (4, 6) im Wesentlichen gemäß einer ersten Ausdehnungsachse (XX') bzw. gemäß einer zweiten Ausdehnungsachse (YY') ausdehnen, wobei die Verteilung der Verankerungsarme (10), die mit dem ersten Teilstück (4) assoziiert sind, betrachtet in einem Regelquerschnitt zur ersten Ausdehnungsachse (XX'), bezogen auf die Verteilung der Verankerungsarme (110), die mit dem zweiten Teil (6) assoziiert sind, betrachtet in einem Regelquerschnitt zur zweiten Ausdehnungsachse (YY'), winkelversetzt ist.

15. Implantat nach Anspruch 14, **dadurch gekennzeichnet, dass** der Winkelversatz (δ) zwischen dem Ring der ersten Verankerungsarme (10) und dem Ring der zweiten Verankerungsarme (110) im Wesentlichen gleich 45 ° ist.

16. Implantat nach Anspruch 12, **dadurch gekennzeichnet, dass** die Gestalt des zweiten Befestigungsmittels (7) mit der des ersten Befestigungsmittels (5) identisch ist.

17. Implantat nach einem der vorhergehenden Ansprüche, **dadurch gekennzeichnet, dass** es in einem Stück (Monoblock) gefertigt ist.

18. Implantat nach einem der vorhergehenden Ansprüche, **dadurch gekennzeichnet, dass** es aus einem röhrenförmigen Element gebildet ist, dessen Wand an ihren Enden derart eingeschnitten ist, dass die Enden des Rohrs in eine Vielzahl von Streifen gespalten sind, die die Verankerungsarme (10) bilden.

19. Implantat nach einem der vorhergehenden Ansprüche, **dadurch gekennzeichnet, dass** das erste und das zweite Teilstück (4, 6) sich im Wesentlichen gemäß einer ersten Ausdehnungsachse (XX') bzw. gemäß einer zweiten Ausdehnungsachse (YY') ausdehnen, wobei das Implantat (1) derart gekrümmt ist, dass der Ablenkwinkel (η) der zweiten Ausdehnungsachse (YY') bezogen auf die erste Ausdehnungsachse (XX') im Wesentlichen im Bereich zwischen 10 ° und 20 ° liegt und im Wesentlichen bevorzugt gleich 15 ° ist.

20. Implantat nach einem der vorhergehenden Ansprüche, **dadurch gekennzeichnet, dass** es ein Greifmittel (18) umfasst, das mit einem Positionierer ausgestattet ist, der angeordnet ist, um den Benutzer über die Ausrichtung des Implantats während des Ergreifens und der Handhabung des Implantats zu informieren.

21. Implantat nach einem der vorhergehenden Ansprüche, **dadurch gekennzeichnet, dass** es ein Implantat zur interphalangealen Arthrodese darstellt.

22. Chirurgischer Kit, **dadurch gekennzeichnet, dass** er umfasst:
- einerseits ein Implantat (1) nach einem der Ansprüche 1 bis 21,
- und andererseits ein chirurgisches Instrument (20), das zur Knochenpräparation bestimmt ist, wobei das Instrument eine Stanze (21) umfasst, die eine Vielzahl von hervorstehenden Elementen (22) aufweist die angeordnet sind, um in dem Knochen durch Klopfen gleichzeitig einen Abdruck zu markieren, der eine Vielzahl von Hohlräumen (23) umfasst, die dazu bestimmt sind, die Verankerungsarme (10) aufzunehmen, wobei die Anordnung der Hohlräume (23) der Anordnung der Verankerungsarme (10) im Wesentlichen paarig zugeordnet ist.

23. Verfahren zur Herstellung eines medizinischen Implantats (1) gemäß einem der Ansprüche 1 bis 21, wobei das Verfahren einen Schritt (a) zur Ausführung des ersten Befestigungsmittels (5) umfasst, in dessen Verlauf ein längliches Stück eingeschnitten wird, um einen Teil des letztgenannten in eine Vielzahl von nicht koplanaren Verankerungsarmen (10), die eine Einheit mit dem länglichen Stück bilden, wobei das Verfahren **dadurch gekennzeichnet ist, dass** der Schritt (a) der Ausführung des ersten Befestigungsmittels einen Subschritt (a2) der Spreizung des divergierenden Abschnitts (10A) umfasst, in dessen Verlauf die Verankerungsarme (10) derart nach außen geknickt werden, dass sie gemäß einer im Wesentlichen kegelförmigen Verteilung divergieren, sowie einen Subschritt (a3) der Bildung des konvergierenden Abschnitts (10B), in dessen Verlauf die freien Enden der Verankerungsarme umgebogen werden, indem sie in Richtung der Ausdehnungsachse des länglichen Stücks geknickt werden.

24. Verfahren zur Herstellung nach Anspruch 23, **dadurch gekennzeichnet, dass** der Subschritt (a3) zur Bildung des konvergierenden Abschnitts es ermöglicht, durch Umknicken eine Krümmung (12) zwischen dem divergierenden Abschnitt (10A) und dem konvergierenden Abschnitt (10B) zu bilden, und **dadurch**, dass dem Subschritt (a3) der Bildung des konvergierenden Abschnitts das Ausführen von Schnittlinien derart vorausgeht, dass das Markieren der Krümmung (12) an der Spitze der Letztgenannten eine auszugsperrende Vorrichtung (16), wie etwa einen Widerhaken, durch Umknicken spontan hervortreten lässt.
